(19) Europäisches Patentamt
European Patent Office
Office européen des brevets

(11) EP 1 311 506 B1

(12) EUROPEAN PATENT SPECIFICATION

(45) Date of publication and mention of the grant of the patent:
**14.11.2007 Bulletin 2007/46**

(21) Application number: **01956751.0**

(22) Date of filing: **03.05.2001**

(51) Int Cl.:
*C07D 455/04* (2006.01)

(86) International application number:
**PCT/IN2001/000097**

(87) International publication number:
**WO 2001/085095 (15.11.2001 Gazette 2001/46)**

# (54) CHIRAL FLUOROQUINOLIZINONE ARGININE SALT FORMS

CHIRALE FLUOROCHINOLIZINON ARGININE SALZE

FORMES DE SELS D'ARGININE DE FLUOROQUINOLONE CHIRALES

(84) Designated Contracting States:
**AT BE CH CY DE DK ES FI FR GB GR IE IT LI LU MC NL PT SE TR**

(30) Priority: **08.05.2000 US 566875**
**17.08.2000 US 640947**
**22.11.2000 WOPCT/IN00/00111**
**09.03.2001 US 802793**

(43) Date of publication of application:
**21.05.2003 Bulletin 2003/21**

(73) Proprietor: **Wockhardt Limited**
**Mumbai 400 051 (IN)**

(72) Inventors:
- **DE SOUZA, Noel John**
  **Chikalthana,**
  **Aurangabad 431 210 (IN)**
- **AGARWAL, Shiv, Kumar**
  **1 P Ext.,**
  **Patparganj,**
  **NEW DELHI - 110092 (IN)**
- **PATEL, Mahesh, Vithalbhai**
  **Chikalthana,**
  **Aurangabad 431 210 (IN)**
- **BHAWSAR, Satish, Baliram**
  **Chikalthana,**
  **Aurangabad 431 210 (IN)**
- **BERI, Rupinder, Kaur**
  **Aundh,**
  **PUNE 7(Maharashtra State) (IN)**
- **YEOLE, Ravindra, Dattatraya**
  **Chikalthana,**
  **Aurangabad 431 210 (IN)**
- **SHETTY, Nitin**
  **Chikalthana,**
  **Aurangabad 431 210 (IN)**

(74) Representative: **Kuhnen & Wacker**
**Patent- und Rechtsanwaltsbüro**
**Postfach 19 64**
**85319 Freising (DE)**

(56) References cited:
**WO-A-00/68229** **US-A- 4 399 134**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

## FIELD OF THE INVENTION

The present invention relates to a novel process for manufacturing arginine salt forms of the chiral fluoroquinolone. More particularly, it relates to a process for preparing arginine salt forms of S-(-)-9-fluoro-6,7-dihydro-8-(4-hydroxypiperidin-1-yl)-5-methyl-1-oxo-1H,5H-benzo[i,j] quinolizine-2-carboxylic acid.

## BACKGROUND OF THE INVENTION

The chiral fluoroquinolone known under the name S-(-)-9-fluoro-6,7-dihydro-8-(4-hydroxypiperidin-1-yl)-5-methyl-1-oxo-1H,5H-benzon|i,j] quinolizine-2-carboxylic acid is described in JP Patent 63,192,753A, JP Patent 05,339,238A, and in our pending U.S. Patent Applications No. 09/566,875 and 09/640,947, WO 00/68229 and PCT Application No. PCT/IN00/00111.

S-(-)-9-fluoro-6,7-dihydro-8-(4-hydroxypiperidin-1-yl)-5-methyl-1-oxo-1H,5H-benzo[i,j] quinolizine-2-carboxylic acid is an optically active isomer of the racemic compound which is claimed in US Patent 4,399,134. Although S-(-)-9-fluoro-6,7-dihydro-8-(4-hydroxypiperidin-1-yl)-5-methyl-1-oxo-1H,5H-benzo[i,j] quinolizine-2-carboxylic acid is not presently an ingredient of any commercial product, it is potentially useful as a commercial antimicrobial agent.

S-(-)-9-fluoro-6,7-dihydro-8-(4-hydroxypiperidin-1-yl)-5-methyl-1-oxo-1H,5B-benzo [i,j] quinolizine-2-carboxylic acid has an aqueous solubility of 0.8 - 2.0 mg/ml over the pH range 8.0 - 9.5 at 28°C, thus creating problems in having to formulate the drug as a tablet or capsule, or in making formulations for gavage and parenteral injection. The need for a salt is dearly indicated, as the lack of an appropriate salt form can hinder the development of dosage forms acceptable for systemic, use in mammals.

S-(-)-9-fluoro-6,7-dihydro-8-(4-hydroxypiperidin-1-yl)-5-methyl-1-oxo-1H,5H-benzo [i,j] quinolizine-2-carboxylic acid has a pKa value of 6.80 suggesting a weak acid character and thus an ability to form a salt with an appropriate base. Generally, conversion of a pharmacologically active compound into a salt form induces a change in the compound's physicochemical properties such as solubility, absorption velocity, etc. Pharmaceutically more desirable salt forms may be selected by studying whether or not a crystalline or amorphous form, or polymorph or pseudopolymorph can be produced, and determining the properties including its physicochemical or biological properties. A pseudopolymorph is a polymorph that differs from a true polymorph by the incorporation of solvent (Solid-state Chemistry of Drugs, 2nd Ed. S.R. Byrn et al (Eds). SSCI, Inc. 1999, p-514).

Pharmaceutically acceptable salts of racemic 9-fluoro-6,7-dihydro-8-(4-hydroxypiperidin-1-yl)-5-methyl-oxo-1H,5H-benzo[i,j] quinolizine-2-carboxylic acid such as salts with inorganic bases and organic bases are mentioned in the text of Otsuka's U.S. Patent No.4,399,134. Besides salts with inorganic bases and organic bases, amino acid salts of S-(-)-9-fluoro-6,7-dihydro-8-(4-hydroxypiperidin-1-yl)-5-methyl-1-oxo-1H,5B-benzo[i,j] quinolizine-2-carboxylic acid are identified in our pending U.S. Patent Applications No. 09/566,875 and U.S. Patent Application No. 09/640,947, WO 00/68229 and PCT Application No. PCT/IN00/00111. The subject matter of these applications is incorporated herein by reference.

S-(-)-9-fluoro-6,7-dihydro-8-(4-hydroxypiperidin-1-yl)-5-methyl-1-oxo-1H,5H-benzo [i,j] quinolizine-2-carboxylic acid, L-arginine salt 0.25 hydrate and S-(-)-9-fluoro-6,7-dihydro-8-(4-hydroxypiperin-1-yl)-5-methyl-1-oxo-1H, 5H-benzo [i,j]quinolizine-2-carboxylic acid, L-arginine salt, 0.75 hydrate described in Examples 7 and 8 of U.S. Patent Applications 09/566,875 and 09/640,947, WO 00/68229 and PCT Application No. PCT/IN00/00111 respectively are highly hygroscopic and turn into syrups on exposure at a relative humidity of 41%. This presents major problems in bulk or manufacturing scale. Many hydrates and salts associated with water are susceptible to changes in humidity, are hygroscopic under adverse storage conditions and during pharmaceutical processing of them to medicament forms.

Amino acid salts, inorganic base and alkali salts and organic base salts of S-(-)-9-fluoro-6,7-dihydro-8-(4-hydrexypiperidin-1-yl)-5-methyl-1-oxo-1H,5H-benzo [i,j] quinolizine-2-carboxylic acid were prepared and studied by the inventors and it was found that:

(a) the arginine salt may exist in either a substantially crystalline form or a substantially amorphous form, each form having distinctive physicochemical, solubility and stability properties;
(b) the arginine salt is less prone than the sodium salt to absorb moisture at specified humidity levels;
(c) the arginine salt, whether crystalline or amorphous, possesses a lower propensity to cause phlebitis than the sodium and potassium salts as determined in rats by intravenous administration; and
(d) the arginine salt is less toxic in rodents than the alkali salt forms.

In summary, the substantially crystalline form and substantially amorphous form of the arginine salt of S-(-)-9-fluoro-6,7-dihydro-8-(4-hydroxypiperidin-1-yl)-5-methyl-1-oxo-1H,5H benzo[i,j]quinolizine-2-carboxylic acid have been

found by the inventors to have very desirable properties in possessing under specified conditions, less hygroscopicity, favourable aqueous solubility, a low propensity to cause phlebitis, and favourable acute toxicity values. These forms are expected to be very useful as pharmaceutical agents as compared with the sodium salt, other inorganic base/alkali salts, organic base salts and other aminoacid salts. These advantages will be apparent from the experimental data shown hereafter.

## SUMMARY OF THE INVENTION

The main objective of the invention accordingly relates to methods for preparing arginine salts of S-(-)-9-fluoro-6,7-dihydro-8-(4-hydroxypiperidin-1-yl)-5-methyl-oxo-1H,5H-benzo [i,j] quinolizine-2-carboxylic acid of the formula I

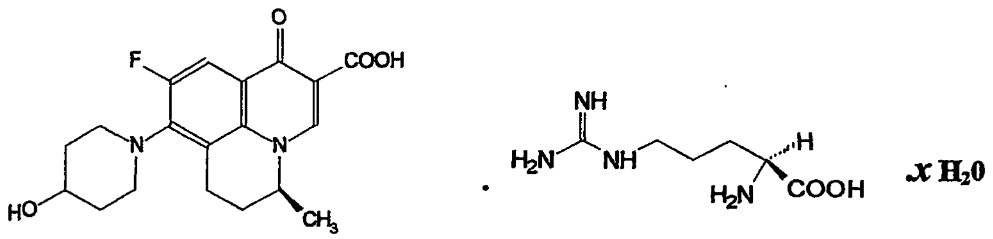

**Formula I**

in which *x* denotes 0.

Still another object of the present invention is to provide a process for bulk scale production of S-(-)-9-fluoro-6,7-dihydro-8-(4-hydroxypiperidin-1-yl)-5-methyl-1-oxo-1H,5H-benzo [i,j] quinonzine-2-carboxylic acid arginine salts of this invention.

## BRIEF DESCRIPTION OF THE DRAWINGS

The invention will now be described in further detail with reference to the accompanying drawings.

FIG. 1 represents the X-Ray Powder Diffraction (XRPD) spectrum of the substantially crystalline form of S-(-)-9-fluoro-6,7-dihydro-8-(4-hydropiperidin-1-yl)-5-methyl-1-oxo-1H,5H-benzo[i,j]quinolizine-2-carboxylic acid arginine salt of the invention.

FIG. 2 represents the X-Ray Powder Diffraction (XRPD) spectrum of the substantially amorphous form of S-(-)-9-fluoro-6,7-dihydro-8-(4-hydroxypiperidin-1-yl)-5-methyl-1-oxo-1H,5H-benzo[i,j]quinolizine-2-carboxylic acid arginine salt of the invention.

FIG. 3 represents the Differential Scanning Calorimeter (DSC) thermogram of the substantially crystalline form of S-(-)-9-fluoro-6,7-dihydro-8-(4-hydroxypiperidin-1-yl)-5-methyl-1-oxo-1H,5H-benzo[i,j]quinolizine-2-carboxylic acid arginine salt of the invention.

FIG. 4 represents the Differential Scanning Calorimeter (DSC) thermogram of the substantially amorphous form of S-(-)-9-fluoro-6,7-dihydro-8-(4-hydroxypiperidin-1-yl)-l5-methyl-1-oxo-1H,5H-benzo[i,j]quinolizine-2-carboxylic acid arginine salt of the invention.

FIG. 5 shows that the particle size measured as mean mass diameter of the substantially crystalline form of S-(-)-9-fluoro-6,7-dihydro-8-(4-hydroxypiperidin-1-yl)-5-methyl-1-oxo-1H,5H-benzo[i,j]quinolizine-2-carboxylic acid arginine salt of the invention is ca. 84 μm and the particle size of the substantially amorphous form of S-(-)-9-fluoro-6,7-dihydro-8-(4-hydroxypiperidin-1-yl)-5-methyl-1-oxo-1H,5H-benzo[i,j] quinolizine-2-carboxylic acid arginine salt of the invention is ca. 32.5 μm.

FIG. 6 shows that the density of the substantially crystalline form of the invention is between 0.51 g/cm$^3$ (untapped) and 0.7 g/cm$^3$ (tapped), and the density of the substantially amorphous form of the invention is between 0.52 g/cm$^3$ (untapped) and 0.7 g/cm$^3$ (tapped).

## DISCLOSURE OF THE INVENTION

The invention accordingly relates to new methods for producing arginine salts of S-(-)-9-fluoro-6,7-dihydro-8-(4-hydroxypiperidin-1-yl)-5-methyl-1-oxo-1H,5H-benzo[i,j]quinolizine-2-carboxylic acid of the formula I

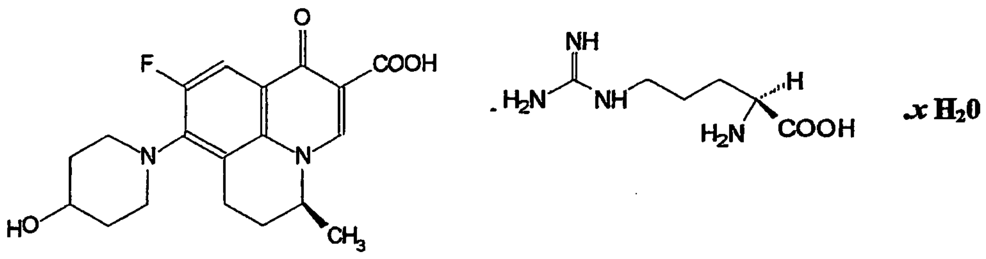

**Formula I**

in which *x* denotes 0.

These salts have favourable aqueous solubility, are stable in humid conditions, have favorable bioavailability, have a lower propensity to cause phlebitis and lower toxicity when administered in mammals.

This invention relates to a process for the preparation of the salts.

A preferred arginine isomer is the S-isomer, that is L-arginine.

The arginine salt produced according to the invention is substantially an anhydrate wherein x = 0. This anhydrate can exist in two polymorphic forms, one described as a substantially amorphous form (hereinafter amorphous form) and the other described as a substantially crystalline form (hereinafter crystalline form). The arginine salts of the process of this invention are described as substantially crystalline or substantially amorphous because the degree of crystallinity of the crystalline form is not 100% and there is a degree of crystallinity in the amorphous form (see Figure 2). Each of the two forms are shown to have distinct physicochemical properties, are more stable to decomposition from uptake of moisture at specified conditions, are distinguished by increased stability, in particular during storage at specified humidities and can be dried without caking or decomposing at elevated temperatures under reduced pressure. Both forms are particularly suitable for the preparation of stable pharmaceutical preparations, and have favourable biological properties.

Both forms, while less hygroscopic than the compounds of Examples 7 and 8 of U.S. Patent Applications 09/566,875 and 09/640,947, WO 00/68229 and PCT Application No. PCT/IN/00/00111, are to a certain degree hygroscopic. In stability studies at varying relative humidity conditions, both forms were found to remain stable at relative humidity values of 22% at 25°C. In stability studies at elevated temperatures, both forms remained stable with no decomposition up to 70°C, however, there was reduction in levels of water content and solvent content. On drying the forms at elevated temperatures over different periods of time and under different humidity conditions, the amounts of water found associated with the salt are altered. However, there is no change in the XRD-patterns of the forms. Without being bound to any theory, from this the inventors infer that the forms are substantially anhydrates and the values of the superficial amounts of associated water are amount due to the hygroscopic nature of the salt and not to the inventors knowledge to any incorporation of water in the lattice structure of the crystal water. The amorphous form tends to be slightly more hygroscopic than the crystalline form.

An anhydrate crystal form is a form that contains no solvent of crystallization (Solid-State Chemistry of Drugs, second edition, Stephen R. Byrn, Ralph R. Pfeiffer and Joseph Stowell, SSCI, Inc. West Lafayette, Indiana). Hydrates are said to exist when the drug substance incorporates water in the crystal lattice in either stochiometric or non-stochiometric amounts" (Stephen Byrn, et. al., Pharmaceutical Solids: A strategic Approach to Regulatory Considerations, Pharma. Res. VoL 12 (7), 1995,945-954).

The inventors have found that S-(-)-9-fluoro-6,7-dihydro-8-(4-hydroxypiperidin-1-yl)-5-methyl-1-oxo-1H,5H-benzo [i,j] quinolizine-2-carboxylic acid arginine salt forms of this invention, have acceptable aqueous solubility of 1 mg/ml extending to 40 mg/ml, depending on the form, over the pH range 8.0 - 9.5 at ambient temperatures. The solubility of the crystalline form is 4.0 to 6.0 mg/ml at pH 9.5. The solubility of the amorphous form is 38.0 to 40.0 mg/ml at pH 9.5. The arginine salt forms are stable on heating at temperatures up to 65 - 70°C at relative humidity ranges up to 22%. They have an acceptable dissolution rate.

The combination of physical properties of the novel S-(-)-9-fluoro-6,7-dihydro-8-(4-hydroxypiperidin-1-yl)-5-methyl-1-oxo-1H,5H-benzo[i,j]quinolizine-2-carboxylic acid arginine salt forms of the present invention with respect to the degree of crystallinity, particle diameter, density, hygroscopicity, water content and content of other solvents are favourable and permit the manufacturing of S-(-)-9-fluoro-6,7-dihydro-8-(4-hydroxypiperidin-1-yl)-5-methyl-1-oxo-1H, 5H-benzo[i,j]quinolizine-2-carboxylic acid arginine salt in a composition which possesses the desired properties.

The properties of the crystalline and amorphous forms including the melting points are of values that endow the forms with desirable compression and flow properties for the processing of dosage forms useful for medicinal purposes.

The crystalline and amorphous forms of S-(-)-9-fluoro-6,7-dihydro-8-(4-hydroxypiperidin-1-yl)-5-methyl-1-oxo-1H,5H-benzo [i,j]quinolizine-2-carboxylic acid arginine salt also have desirable physicochemical properties as hereinbefore mentioned, and are characterised by parameters such as XRD, DSC, particle size and powder density.

The substantially crystalline form is described below:

(a) The degree of crystallinity as determined by X-ray powder diffraction is shown in Fig. 1, X-ray powder diffraction (2θ): 10.16, 11.78, 12.52, 16.00, 18.94, 19.66, 20.36, 21.28, 21.92,22.52,24.74,25.28,30.74.
(b) A thermogram as determined by Differential scanning calorimetry is shown in Fig. 3 (DSC exotherm at 242.18°C (onset at 207.85°C)).

It is desirable that the substantially crystalline form also has the following properties:

(c) Particle size measured as mean mass diameter (MMD) less than 99 μm, preferably less than 85 μm as determined by laser diffraction technique, and as shown in Fig. 5;
(d) Density between 0.51 g/cm$^3$ (untapped) and 0.7 g/cm$^3$ (tapped) As shown in Fig. 6;
(e) Hygroscopicity not exceeding 0.1 % increase of weight upon storage for 14 days up to 22% relative atmospheric humidity as determined gravimetrically;
(f) A content of water between 0.1 and 0.2% by weight as determined by titration according to Karl Fischer; and
(g) When methyl ethylketone, methylisobutylketone or acetone is used in the preparation of this form, the content of the solvent is less than 0.5 %, preferably less than 0.4 % by weight as determined by gas chromatography. When acetonitrile is used as solvent, the content of acetonitrile is less than 0.04 %, preferably less than 0.03 % by weight as determined by gas chromatography.

The substantially amorphous form is described below:

(a) The degree of crystallinity as determined by X-ray powder diffraction is shown in Fig. 2, X-ray powder diffraction (2θ): 18.28, 18.8, 19.8, 20.12, 20.62, 21.10, 21.44, 21.88, 22.6, 23.02.
(b) A thermogram as determined by Differential scanning calorimetry is shown in Fig. 4 (DSC endotherm at 194.60°C (onset at 185.28°C) and an exotherm at 245.81°C (onset at 226.23°C)).

It is desirable that the substantially amorphous form also has the following properties:

(c) Particle size measured as mean mass diameter (MMD) less than 43 μm, preferably less than 34 μm as determined by laser diffraction technique, and as shown in Fig. 5;
(d) Density between 0.52 g/cm$^3$ (untapped) and 0.7 g/cm$^3$ (tapped) as shown in Fig. 6;
(e) Hygroscopicity not exceeding 0.1 % increase of weight upon storage for 14 days up to 22% relative atmospheric humidity as determined gravimetrically;
(f) A content of water between 0.4 and 0.7% by weight as determined by titration according to Karl Fischer; and
(g) When an alkanol is used as the solvent in the preparation of the substantially amorphous form, the content of solvent is less than 0.3% by weight, preferably less than 0.2% by weight as determined by gas chromatography.

The S-(-)-9-fluoro-6,7-dihydro-8-(4-hydroxypiperidin-1-yl)-5-methyl-1-oxo-1H,5H-benzo [i,j]quinolizine-2-carboxylic acid arginine salt exhibits the same antibacterial activity as the free active ingredient, S-(-)-9-fluoro-6,7-dihydro-8-(4-hydroxypiperidin-1-yl)-5-methyl-1-oxo-1H,5H-benzo[i,j]quinolizine-2-carboxylic acid.

The substantially crystalline and substantially amorphous forms of S-(-)-9-fluoro-6,7-dihydro-8-(4-hydroxypiperidin-1-yl)-5-methyl-1-oxo-1H,5H-benzo[i,j]-quinolizine-2-carboxylic acid arginine salt anhydrate have antimicrobial activity:

They have potent activity against resistant *Staphylococcus aureus.*

They have ability to resist drug efflux in Gram-positive organisms.

They have the unusual ability to retain potency at an acidic pH of 5.5.

They are effective in treating respiratory pathogens.

The are effective in treating resistant mutants.

They have superior cidal action against slow-growing *Staphylococci*.

They score over the standard drugs in terms of eradication efficacy for *Staphylococci* from vital organs and thigh muscle.

The present invention therefore relates to a process for preparing the novel polymorphic forms of the arginine salt. A process for the manufacture of the substantially crystalline and substantially amorphous forms of S-(-)-9-fluoro-6,7-dihydro-8-(4-hydroxypiperidin-1-yl)-5-methyl-1-oxo-1H,5H-benzo[i,j]quinolizine-2-carboxylic acid arginine salt comprises the following consecutive steps:

a) treating S-(-)-9-fluoro-6,7-dihydro-8-(4-hydroxypiperidin-1-yl)-5-methyl-1-oxo-1H, 5H-benzo[i,j]quinolizine-2-carboxylic acid with arginine in a solvent mixture composed of water and an organic solvent, to form a solution;
b) concentrating the solution under reduced pressure to provide a residue;
c) treating the residue with an alkane;
d) isolating the substantially crystalline or substantially amorphous form of S-(-)-9-fluoro-6,7-dihydro-8-(4-hydrozypiperidin-1-yl)-5-methyl-1-oxo-1H, 5H-benzo[i,j]quinolizine-2-carboxylic acid arginine salt; and
e) purifying and drying the substantially crystalline or substantially amorphous form of S-(-)-9-fluoro-6,7-dihydro-8-(4-hydroxypiperidin-1-yl)-5-methyl-1-oxo-1H, 5H-benzo [i,j] quinolizine-2-carboxylic acid arginine salt using conventional methods.

Preferably the ratio of arginine to S-(-)-9-fluoro-6,7-dihydro-8-(4-hydroxypiperidin-1-yl)-5-methyl-1-oxo-1H, 5H, benzo [i,j] quinolizine-2-carboxylic acid is 1 mole to 1 mole.

Preferably the solvent mixture used in step (a) is composed of water and an organic solvent. When the organic solvent is a ketone or nitrile, the content of water in the solvent mixture is 20% - 50%. When the organic solvent is methanol, the content of water in the solvent mixture is 2% - 10%; when the organic solvent is ethanol, the water content in the solvent mixture is about 15%; when the organic solvent is isopropanol, the water content in the solvent mixture is about 50%.

Preferably the solution of step (a) is stirred for 30-60 minutes.

Preferably in step (b) the solution is concentrated at 170-180 m bar and then at 40-60 m bar.

In a preferred embodiment, the aqueous solution of arginine is added to a solution of an equimolar amount of S-(-)-9-fluoro-6,7-dihydro-8-(4-hydroxypiperidin-1-yl)-5-methyl-1-oxo-1H,5H-benzo[i,j]quinolizine-2-carboxylic acid in an organic solvent, stirred for 30 - 60 minutes, and concentrated under reduced pressure, first at 170 -180 mbar and then at 40 - 60 mbar to provide a residue. Organic solvents may be selected from ($C_1$-$C_6$) ketones, ($C_1$-$C_6$) nitriles, or ($C_1$-$C_6$) alkanols and the like. When the organic solvent used is a ketone or nitrile such as methyethylketone, methylisobutylketone, methylbutylketone, acetone or acetonitrile, the residue obtained is substantially crystalline. When the organic solvent used is one such as an alkanol, for example methanol, ethanol or isopropanol, the residue obtained is substantially amorphous. The residue is treated with an alkane such as a ($C_1$-$C_6$) alkane, preferably hexane or pentane. The mixture is stirred for 3 - 5 hours, and the desired salt isolated by filtration or centrifugation, washed if necessary with additional amounts of alkane, preferably hexane or pentane, and dried preferably under reduced pressure and heating up to 65 - 70° C.

The process for manufacturing the new forms of S-(-)-9-fluoro-6,7-dihydro-8-(4-hydroxypiperidin-1-yl)-5-methyl-1-oxo-1H, 5H-benzo[i,j]quinolizine-2-carboxylic acid arginine salt differs from the earlier known processes in that it can be controlled to give two different polymorphic forms of the substantially anhydrate salt form through use of the respective solvents that are shown to result in the two forms. This process can be carried out in conventional chemical process equipment and produces a product, which is substantially an anhydrate. It was not possible to obtain these products with known processes.

A comparison between the different samples of the novel forms of the arginine salt prepared by the process of the present invention and those obtained from the experiments disclosed in the prior art shows that the salt of the present invention is substantially an anhydrate, is stable up to 70°C and at relative humidity up to 22% at 25°C, and has favourable aqueous solubility, all attributes essential to the preparation of pharmaceutical compositions. The novel forms of S-(-)-9-fluoro-6,7-dihydro-8-(4-hydroxypiperidin-1-yl)-5-methyl-1-oxo-1H,5H-benzo[i,j]quinolizine-2-carboxylic acid arginine salt are also stable under typical storage conditions, have good bioavailability in mammals, have lower phlebitis-forming potential on administration to mammals, have low or reduced toxicity, have acceptable disintegration and dissolution rates, and hence are very useful for pharmaceutical manufacturing and for use in medicine. The forms are specially suitable for long-term intravenous therapy in critically ill patients or patients in intensive care units. Injectable preparations of the arginine salt can be readily prepared in view of its availability in a bulk form that remains stable under specified conditions, its favourable aqueous solubility, its ideal suitability in not causing venous inflammation on repeated intravenous administration, and its safety from adverse toxicity.

An S-(-)-9-fluoro-6,7-dihydro-8-(4-hydroxypiperidin-1-yl)-5-methyl-1-oxo-1H,5H-benzo [i,j]quinolizine-2-carboxylic acid arginine salt form of this invention can be present in pharmaceutical formulations as the only active compound

or can be combined with other active ingredients such as other antibacterial agents.

The invention therefore also relates to liquid and solid pharmaceutical formulations which comprise the arginine salt of the invention, such as for example, injectable solutions, suspensions, emulsions, tablets, coated tablets, coated tablet cores, capsules, solutions, troches, dispersions, patches, powders, lotions, gels, sprays, pellets, granules, suppositories, hard or soft gelatin capsules, ointments, creams and the like.

The pharmaceutical formulations are prepared in a manner known per se, for example by mixing, stirring, suspending, dispersing, emulsifying, dissolving and the like, the active compounds with or in the pharmaceutical auxiliaries such as a carrier, diluent, solvent or excipient and processing the components to pharmaceutically suitable forms for parenteral, oral, topical, intranasal, buccal or rectal administration and the like.

Pharmaceutical formulations can be formulated together with auxiliaries and additives usually employed in pharmacy, such as tablet binders, fillers, preservatives, tablet disintegrating agents, flow regulating, agents, plasticizers, wetting agents, dispersing agents, emulsifiers, solvents, pH altering additives, flavourings and the like.

The total daily dose range is generally from about 200mg to about 1500mg of the arginine salt form. However, the dose may be higher or lower depending on the needs and conditions of the patient.

The following detailed examples serve to more fully illustrate the invention without limiting its scope.

EXAMPLE 1

S-(-)-9-Fluoro-6,7-dihydro-8-(4-hidroxypiperin-1-yl)-5-methyl-1-oxo-1H,5H-benzo [i,j] quinolizine-2-carboxylic acid arginine salt Synthesis of Substantially Crystalline Product

A solution of L-(+)-arginine (48.372 g, 0.278 mole) in distilled water (600 ml) was added dropwise over a period of 30 min to the stirred solution/suspension of finely powdered S-(-)-9-fluoro-6,7-dihydro-8-(4-hydroxypiperidin-1-yl)-5-methyl-1-oxo-1H,5H-benzo **[i,j]** quinolizine-2-carboxylic acid (100 g, 0.278 mole) in acetone (1250 ml). The obtained dear solution was stirred for 30 min and concentrated on a water bath in vacuum (175 mbar) at 80°C. When product started solidifying, the concentration was carried out in vacuum (50 mbar) at 80°C up to dryness. Hexane (1 liter) was added, the reaction mixture was stirred for 4 hr, the solid thus separated was filtered and dried in vacuum (0.7 mbar) for 12 hrs at 70°C. Yield 145 g (96.9%), m.p. 238-242 °C, and solubility 6 mg/ml (pH 9.5 buffer solution).

The substantially crystalline S-(-)-9-fluoro-6,7-dihydro-8-(4-hydroxypiperidin-1-yl)-5-methyl-1-oxo-1H,5H-benzo[i,j]quinolizine-2-carboxylic acid arginine salt prepared according to Example 1 possesses the following properties:

a) Crystalline form, with a degree of crystallinity as determined by X-ray powder diffraction and as shown in Fig. 1.
b) A thermogram as determined by Differential scanning calorimetry and as shown in Fig. 3.
c) Particle size measured as mean mass diameter (MMD) of 83.92 μm, as determined by laser diffraction technique.
d) Density of 0.51 $g/cm^3$ (untapped) and 0.7 $g/cm^3$ (tapped).
e) Hygroscopicity of 0% increase of weight upon storage for 14 days up to 22% relative atmospheric humidity as determined gravimetrically.
f) A content of moisture water of 0.1 % by weight as determined by titration according to Karl Fischer.
g) A content of acetone of 0.014 % by weight as determined by gas chromatography.

EXAMPLE 2

Similarly, when acetonitrile was used in place of acetone in the process described in Example 1, and the amount of water in the resulting acetonitrile-water solution was about 40%, a substantially crystalline product was obtained. Yield 87%.

EXAMPLE 3

Synthesis of Substantially Amorphous product

When methanol was used in place of acetone in the process described in Example 1, and the amount of water in the resulting methanol-water solution was between 2% to 10%, a substantially amorphous product was obtained. Yield 97.3 %, m.p. 238 - 244 °C, $[\alpha]_D^{25}$-166.2 °(c =1, methanol), and solubility 40 mg/ml in pH 9.5 buffer solution.

The novel form of the substantially amorphous S-(-)-9-fluoro-6,7-dihydro-8-(4-hydroxypiperidin-1-yl)-5-methyl-1-oxo-1H,5B-benzo[i,j]quinolizine-2-carboxylic acid arginine salt according to Example 3 possesses the following properties:

a) Amorphous form, with a degree of crystallinity as determined by X-ray powder diffraction and as shown in Fig. 2.

b) A thermogram as determined by Differential scanning calorimetry and shown in Fig. 4.
c) Particle size measured as mean mass diameter (MMD) of 32.44 μm, as determined by laser diffraction technique.
d) Density of 0.52 g/cm$^3$ (untapped) and 0.7 g/cm$^3$ (tapped).
e) Hygroscopicity of 0% increase of weight upon storage for 14 days up to 22% relative atmospheric humidity as determined gravimetrically.
f) A content of moisture water of 0.41 % by weight as determined by titration according to Karl Fischer.
g) A content of methanol of 0.16 % by weight as determined by gas chromatography.

EXAMPLE 4

Substantially amorphous product was also obtained, when ethanol was used in place of acetone in the process described in Example 1 and the amount of water in the resulting ethanol-water solution was 13 -15 %. Yield 97.44 %.

EXAMPLE 5

Similarly, when isopropanol was used in place of acetone as described in the process of Example 1, and the amount of water in the resulting isopropanol-water solution was about 50%, a substantially amorphous product was obtained. Yield 80 %.

EXAMPLE 6

TEST EXAMPLE

X-ray Diffraction Analysis

300 mg each of S-(-)-9-fluoro-6,7-dihydro-8-(4-hydroxypiperidin-1-yl)-5-methyl-1-oxo-1H,5H-benzo [I,j]quinolizine-2-carboxylic acid L-(+)arginine salt prepared as in Examples 1 and 5 were thinly spread on a sample holder. X-ray diffraction analyses (40kv x 40 mA Rigaku D/max 2200) were performed under the conditions listed below:

Scan speed 5°/ min
Sampling time 7 min
Scan mode: continuous
2θ/θ reflection
Cu target (Ni filter)

Results of the X-ray diffraction analysis on the substantially crystalline and substantially amorphous forms are depicted in Fig. 1 and Fig. 2 respectively. From these spectra it can be verified that their crystal forms differ from each other.

EXAMPLE 7

TEST EXAMPLE

Thermal Analysis of the S-(-)-9-fluoro-6,7-dihydro-8-(4-hydroxypiperidin-1-yl)-5-methyl-1-oxo-1H,5H-benzo [I, j]quinolizine-2-carboxylic acid L-(+)-arginine salts prepared as in Examples 1 and 5.

For the Differential Scanning Calorimetry, METTLER TOLEDO STAR system was used. 5.4 mg of the sample was weighed into the aluminum pan, which was then press sealed with an aluminium lid. After three tiny needle holes were made on the lid the sample was tested by heating from (30°C) to (300°C) at a rate of 10°C/min. As can be seen from Fig. 4 (amorphous form) there is an endothermic peak which begins at around 185°C to 200°C and an exothermic peak due to thermal decomposition at around 226°C to 251°C. In contrast the substantially crystalline form (Fig. 3) shows only an exothermic peak at around 208°C to 256°C without any endothermic peak.

EXAMPLE 8

TEST EXAMPLE

BULK DENSITY DETERMINATION

For untapped and tapped density determination Bulk Density Apparatus (R.V. Electronics, Mumbai) was used.

25 g of the sample was slowly poured into a dry dean stoppered measuring cylinder and filled volume was measured to obtain untapped density.

$$\text{Untapped density} = 25\ \text{g/untapped volume.}$$

Then the measuring cylinder was fixed to Bulk Density Apparatus and sample was tapped (150 times) and volume was measured to furnish the tapped volume.

$$\text{Tapped density} = 25\ \text{g/Tapped volume.}$$

EXAMPLE 9

TEST EXAMPLE

MEAN MASS DETERMINATION

For mean mass diameter Master Sizer of Malvern Instrument LTD U.K. was used under the conditions listed below:

Lens/Focus: 300 mm
Solvent: Hexane
Analysis model: Polydisperse
Obstruction value: 20-23%
Results are depicted in Fig. 5.

**Claims**

1. A process for the manufacture of S-(-)-9-fluoro-6,7-dihydro-8-(4-hydroxypiperidin-1-yl)-5-methyl-1-oxo-1H,5H-benzo[i,j]quinolizine-2-carboxylic acid arginine salt comprising the following consecutive steps:

    a) treating S-(-)-9-fluoro-6,7-dihydro-8-(4-hydroxypiperidin-1-yl)-5-methyl-1-oxo-1H,5H-benzo[i,j]quinolizine-2-carboxylic acid with arginine in a solvent mixture composed of water and an organic solvent to form a solution;
    b) concentrating the solution under reduced pressure to provide a residue;
    c) treating the residue with an alkane;
    d) isolating the crystalline or amorpous S-(-)-9-fluoro-6,7-dihydro-8-(4-hydroxypiperidin-1-yl)-5-methyl-1-oxo-1H,5H-benzo[i,j]quinolizine-2-carboxylic acid arginine salt; and
    e) purifying and drying the crystalline or amorphous form of S-(-)-9-fluoro-6,7-dihydro-8-(4-hydroxypiperidin-1-yl)-5-methyl-1-oxo-1H,5H-benzo[i,j]quinolizine-2-carboxylic acid arginine salt.

2. The process according to claim 1, wherein the ratio of S-(-)-9-fluoro-6,7-dihydro-8-(4-hydroxypiperidin-1-yl)-5-methyl-1-oxo-1H,5H-benzo[i,j]quinolizine-2-carboxylic acid to arginine is 1 mole to 1 mole.

3. The process according to claim 1 or 2, wherein the organic solvent is selected from the group consisting of ($C_1$-$C_6$) ketones, ($C_1$-$C_6$) nitriles, and ($C_1$-$C_6$) alkanols, in particular from the group consisting of methylethylketone, methylisobutylketone, acetone, acetonitrile, methanol, ethanol and isopropanol.

4. The process according to any of claims 1 to 3, wherein the content of water in the solvent mixture is 20 to 50 % when the organic solvent is a ketone or a nitrile, 2 to 10 % when the organic solvent is methanol, 15 % when the organic solvent is ethanol, and 50 % when the organic solvent is isopropanol.

5. The process according to any of claims 1 to 4, wherein the solution of step a) is stirred for 30 to 60 minutes.

6. The process according to any of claims 1 to 5, wherein in step b) the solution is concentrated at 170 to 180 mbar

and then at 40 to 60 mbar.

7. The process according to any of claims 1 to 6, wherein the alkane in step c) is pentane or hexane and the treatment comprises the steps of stirring for 3 to 5 hours, isolating the desired salt by filtration or centrifugation, optionally washing with additional amounts of alkane, and drying under reduced pressure and heating up to 65 to 70° C.

## Patentansprüche

1. Verfahren zum Herstellen von S-(-)-9-Fluor-6,7-dihydro-8-(4-hydroxypiperidin-1-yl)-5-methyl-1-oxo-1H,5H-benzo[i,j]-chinolizin-2-Carbonsäure-Argininsalz umfassend die folgenden aufeinander folgenden Schritte:

   a) Behandeln von S-(-)-9-Fluor-6,7-dihydro-8-(4-hydroxypiperidin-1-yl)-5-methyl-1-oxo-1H,5H-benzo[i,j]-chinolizin-2-Carbonsäure mit Arginin in einer aus Wasser und einem organischen Lösungsmittel bestehenden Lösungsmittelmischung, um eine Lösung zu bilden;
   b) Konzentrieren der Lösung unter vermindertem Druck zum Bereitstellen eines Rückstands;
   c) Behandeln des Rückstands mit einem Alkan;
   d) Isolieren des kristallinen oder amorphen S-(-)-9-Fluor-6,7-dihydro-8-(4-hydroxypiperidin-1-yl)-5-methyl-1-oxo-1 H,5H-benzo[i,j]-chinolizin-2-Carbonsäure-Argininsalzes; und
   e) Reinigen und Trocknen der kristallinen oder amorphen Form von S-(-)-9-Fluor-6,7-dihydro-8-(4-hydroxypiperidin-1-yl)-5-methyl-1-oxo-1H,5H-benzo[i,j]-chinolizin-2-Carbonsäure Argininsalz.

2. Verfahren nach Anspruch 1, worin das Verhältnis von S-(-)-9-Fluor-6,7-dihydro-8-(4-hydroxypiperidin-1-yl)-5-methyl-1-oxo-1H,5H-benzo[i,j]-chinolizin-2-Carbonsäure zu Arginin 1 Mol zu 1 Mol beträgt.

3. Verfahren nach Anspruch 1 oder 2, worin das organische Lösungsmittel ausgewählt ist aus der Gruppe, bestehend aus ($C_1$-$C_6$)-Ketonen, ($C_1$-$C_6$)-Nitrilen und ($C_1$-$C_6$)-Alkanolen, insbesondere aus der Gruppe, bestehend aus Methylethylketon, Methylisobutylketon, Aceton, Acetonitril, Methanol, Ethanol und Isopropanol.

4. Verfahren nach einem der Ansprüche 1 bis 3, worin der Wassergehalt in der Lösungsmittelmischung 20 bis 50% beträgt, wenn das organische Lösungsmittel ein Keton oder ein Nitril ist, 2 bis 10% wenn das organische Lösungsmittel Methanol ist, 15% wenn das organische Lösungsmittel Ethanol ist und 50% wenn das organische Lösungsmittel Isopropanol ist.

5. Verfahren nach einem der Ansprüche 1-4, worin die Lösung aus Schritt a) für 30 bis 60 Minuten gerührt wird.

6. Verfahren nach einem der Ansprüche 1 bis 5, worin in Schritt b) die Lösung bei 170 bis 180 mbar konzentriert wird und dann bei 40 bis 60 mbar.

7. Verfahren nach einem der Ansprüche 1 bis 6, worin das Alkan in Schritt c) Pentan oder Hexan ist und das Behandeln die Schritte des Rührens für 3 bis 5 Stunden, des Isolierens des gewünschten Salzes durch Filtrieren oder Zentrifugieren, optional des Waschens mit zusätzlichen Mengen an Alkan und des Trocknens unter vermindertem Druck und Erwärmen bis zu 65 bis 70°C umfasst.

## Revendications

1. Procédé de fabrication du sel d'arginine de l'acide S-(-)-9-fluoro-6,7-dihydro-8-(4-hydroxypipéridin-1-yl)-5-méthyl-1-oxo-1H,5H-benzo[i,j]-quinolizine-2-carboxylique comprenant les étapes consécutives consistant à :

   a) traiter l'acide S-(-)-9-fluoro-6,7-dihydro-8-(4-hydroxypipéridin-1-yl)-5-méthyl-1-oxo-1H,5H-benzo[i,j]quinolizine-2-carboxylique avec l'arginine dans un mélange solvanté, composé d'eau et d'un solvant organique, pour former une solution ;
   (b) concentrer la solution sous pression réduite pour donner un résidu ;
   (c) traiter le résidu avec un alcane ;
   (d) isoler le sel d'arginine de l'acide S-(-)-9-fluoro-6,7-dihydro-8-(4-hydroxypipéridin-1-yl)-5-méthyl-1-oxo-1H, 5H-benzo[i,j]quinolizine-2-carboxylique à l'état amorphe ou cristallin ; et
   e) purifier et sécher le sel d'arginine de l'acide S-(-)-9-fluoro-6,7-dihydro-8-(4-hydroxypipéridin-1-yl)-5-méthyl-1-oxo-1H,5H-benzo[i,j]quinolizine-2-carboxylique à l'état amorphe ou cristallin.

**2.** Procédé selon la revendication 1, dans lequel le rapport acide S-(-)-9-fluoro-6,7-dihydro-8-(4-hydroxypipéridin-1-yl)-5-méthyl-1-oxo-1H,5H-benzo[i,j]-quinolizine-2-carboxylique/arginine est de 1/1 (mole/mole).

**3.** Procédé selon la revendication 1 ou 2, dans lequel le solvant organique est choisi dans le groupe comprenant les cétones en $C_1$-$C_6$, les nitriles en $C_1$-$C_6$, et les alcanols en $C_1$-$C_6$, en particulier dans le groupe comprenant la méthyle éthyle cétone, la méthyle isobutyle cétone, l'acétone, l'acétonitrile, le méthanol, l'éthanol et l'isopropanol.

**4.** Procédé selon l'une quelconque des revendications 1 à 3, dans lequel la teneur en eau dans le mélange solvanté a une valeur allant de 20 à 50 % quand le solvant organique est une cétone ou un nitrile, allant de 2 à 10 % quand le solvant organique est le méthanol, de 15 % quand le solvant organique est l'éthanol, et de 50 % quand le solvant organique est l'isopropanol.

**5.** Procédé selon l'une quelconque des revendications 1 à 4, dans lequel la solution de l'étape a) est mélangée pendant 30 à 60 minutes.

**6.** Procédé selon l'une quelconque des revendications 1 à 5, dans lequel dans l'étape b) la solution est concentrée sous une pression allant de 170 à 180 mbar et ensuite de 40 à 60 mbar.

**7.** Procédé selon l'une quelconque des revendications 1 à 6, dans lequel l'alcane dans l'étape c) est le pentane ou l'hexane et le traitement comprend les étapes consistant à mélanger pendant 3 à 5 heures, isoler le sel désiré par filtration ou centrifugation, de manière facultative laver avec des quantités supplémentaires d'alcane, et sécher sous pression réduite et chauffer jusqu'à une température de 65 à 70 °C.

NDD/PCT/005

FIG.1

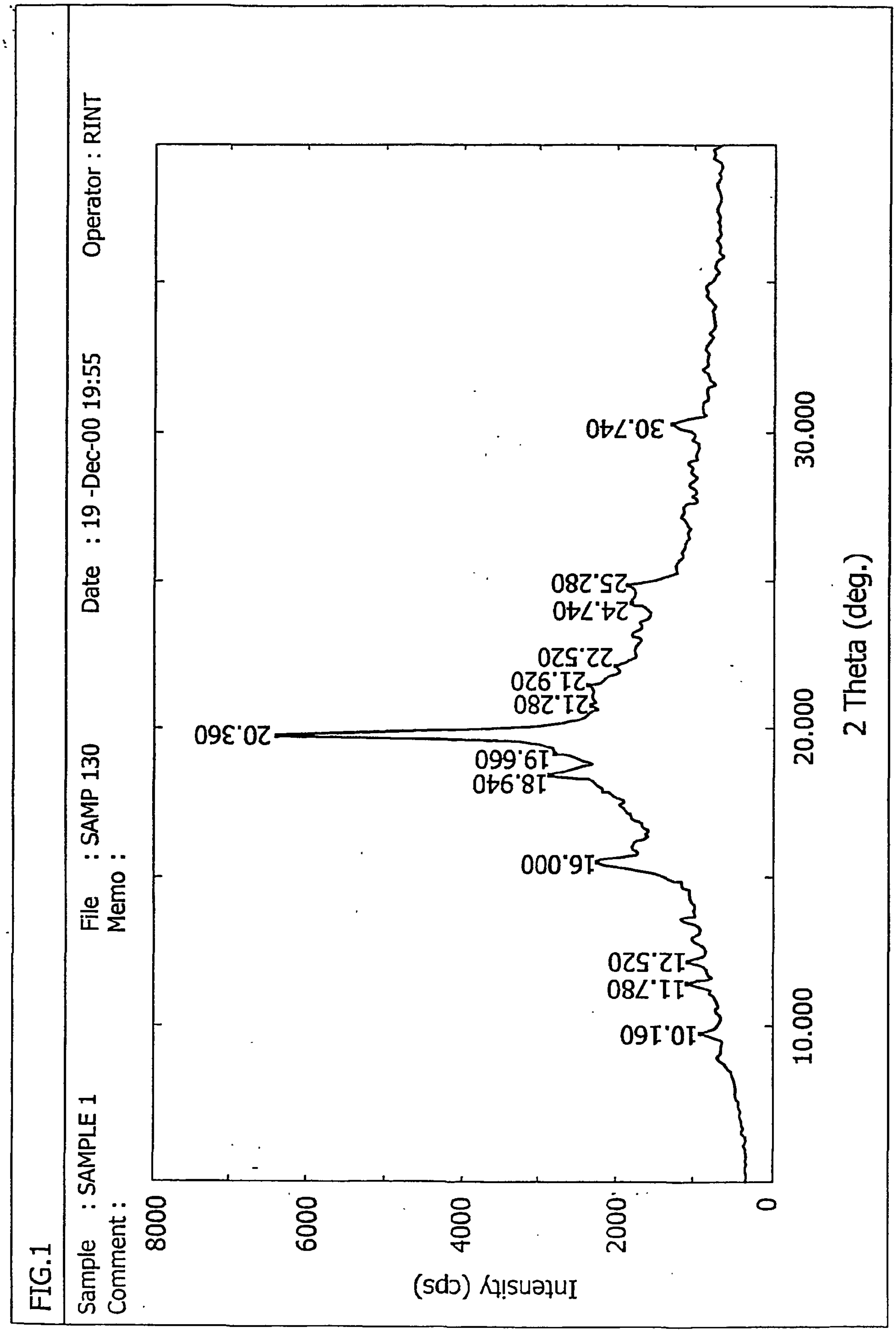

NDD/PCT/005

FIG. 2

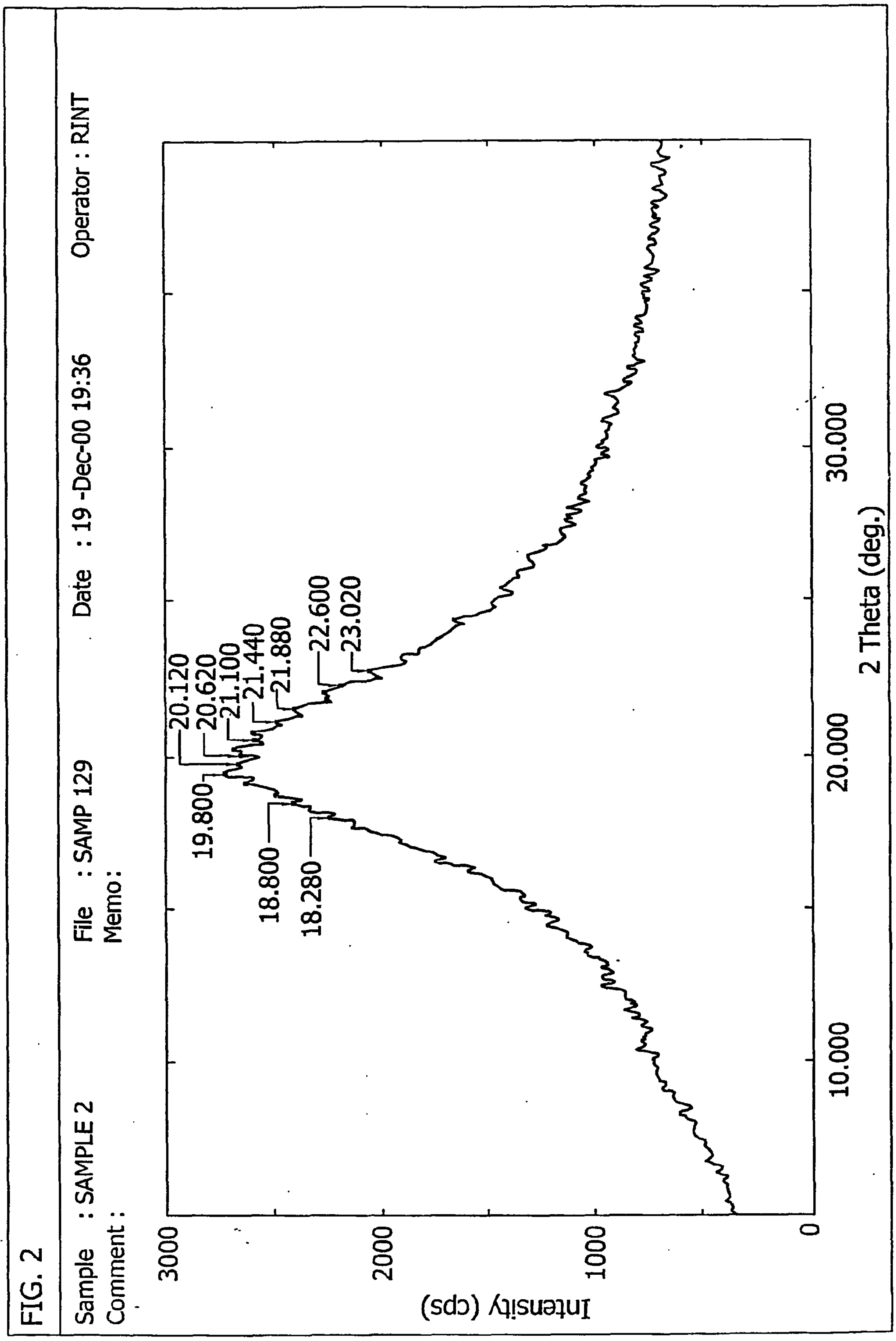

NDD/PCT/005

FIG. 3

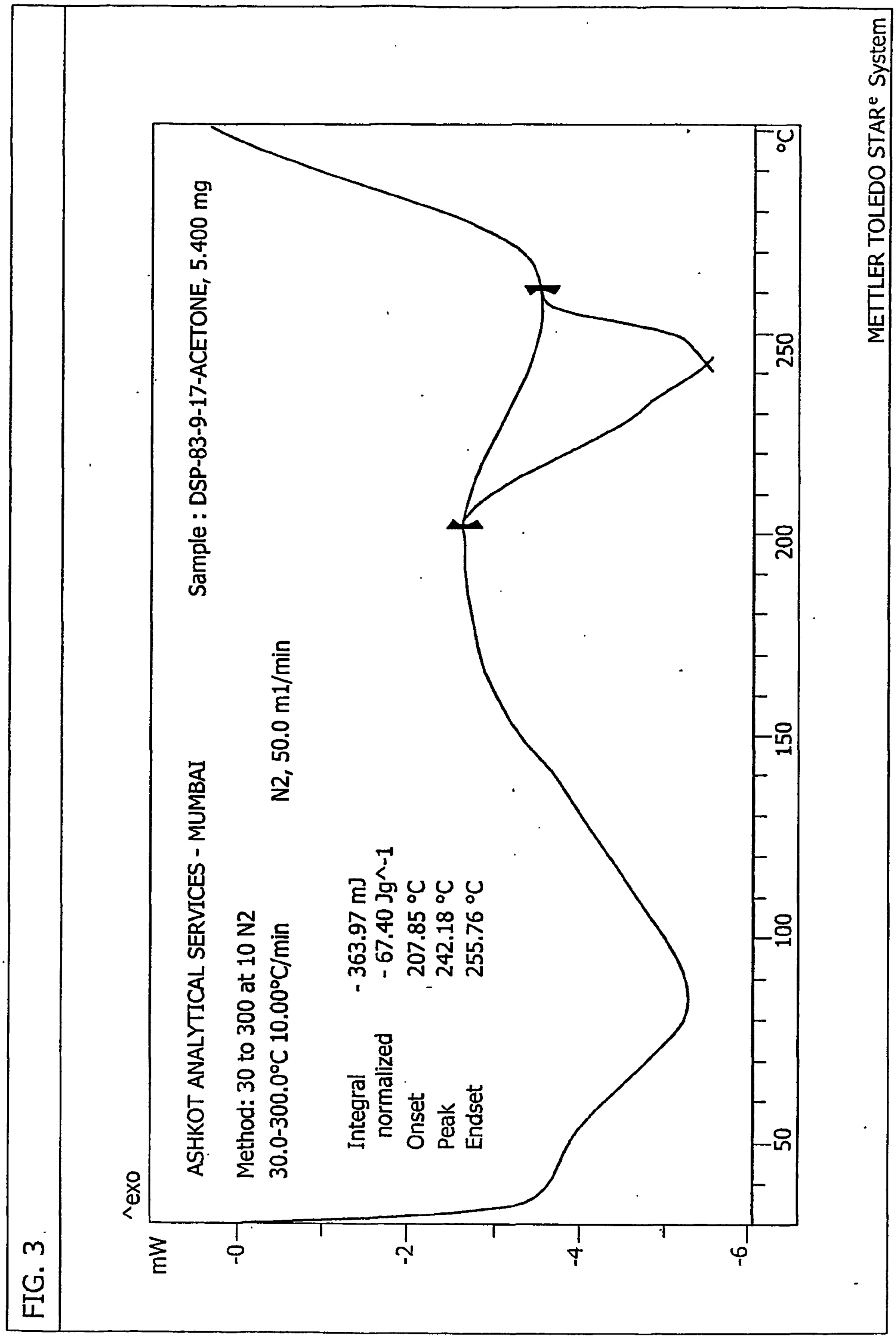

NDD/PCT/005

FIG. 4

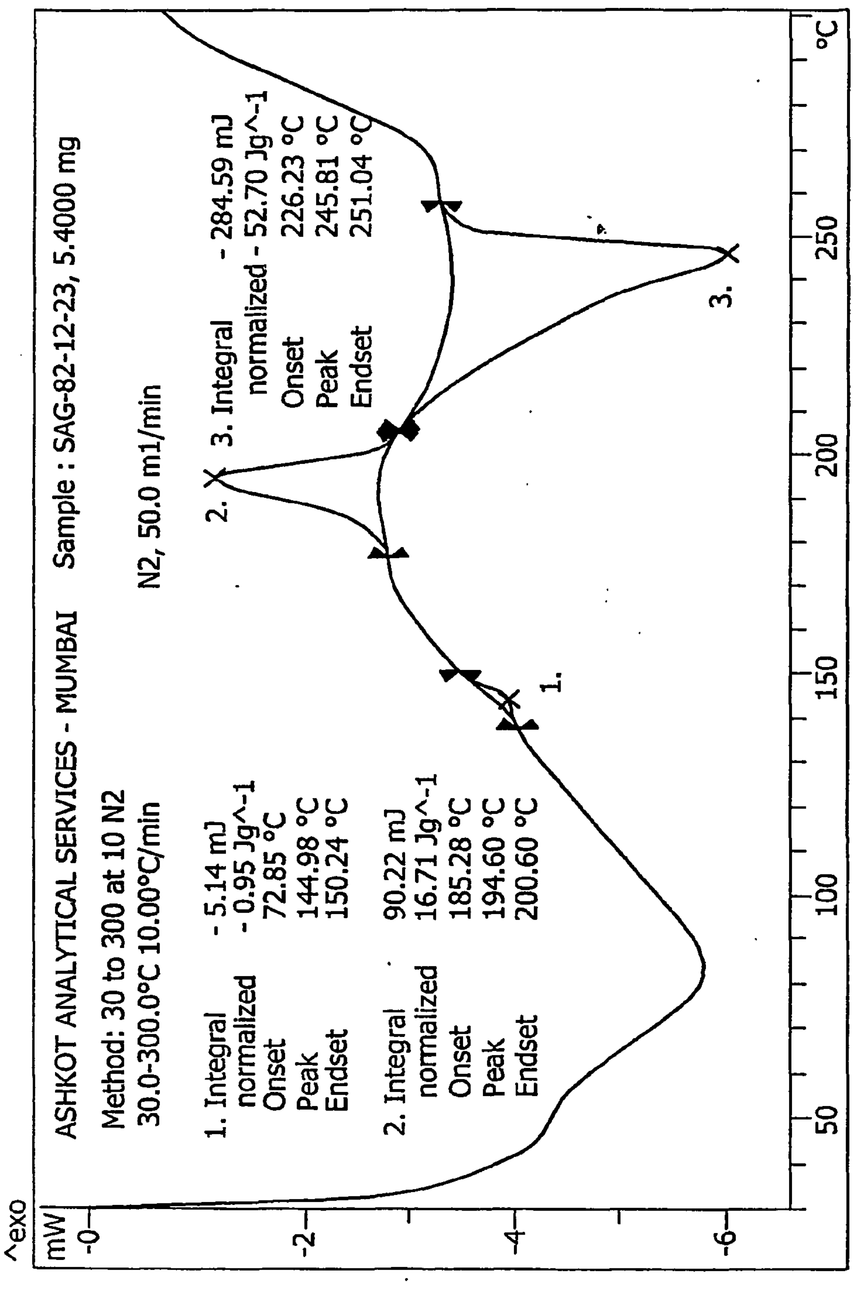


METTLER TOLEDO STAR[e] System

NDD/PCT/005

FIG. 5

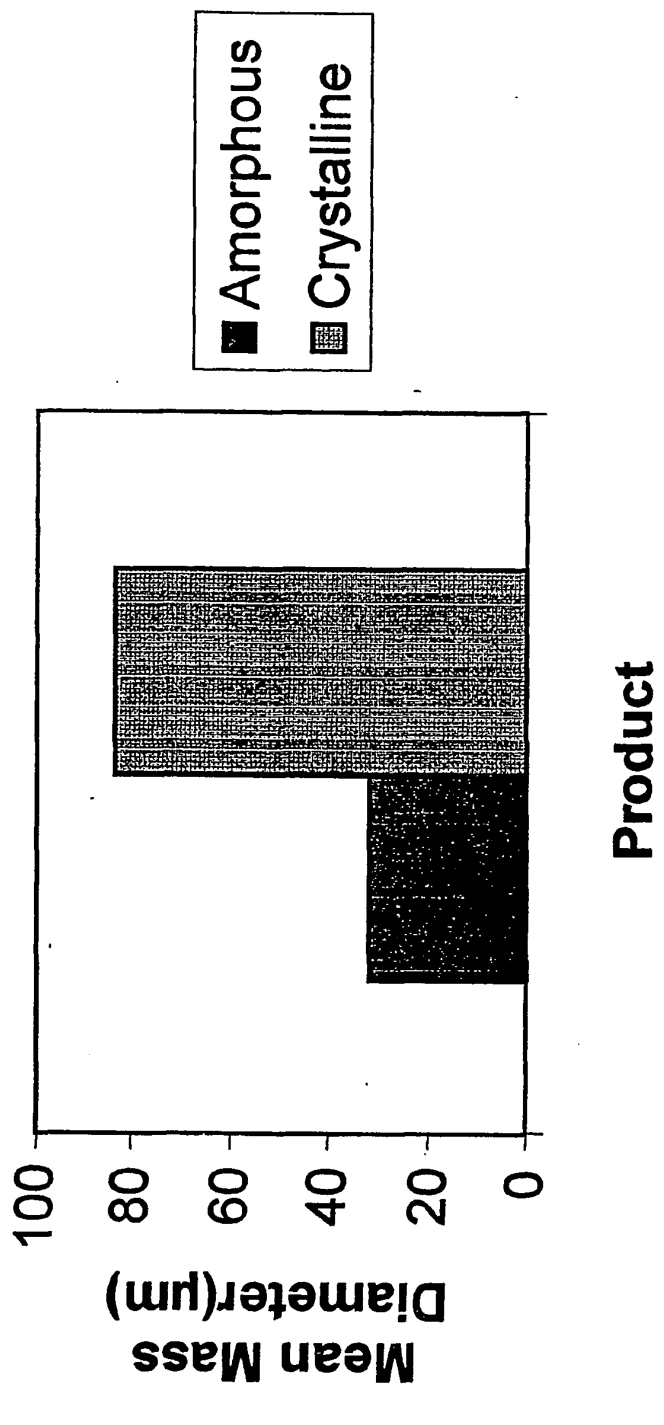

NDD/PCT/005

FIG. 6

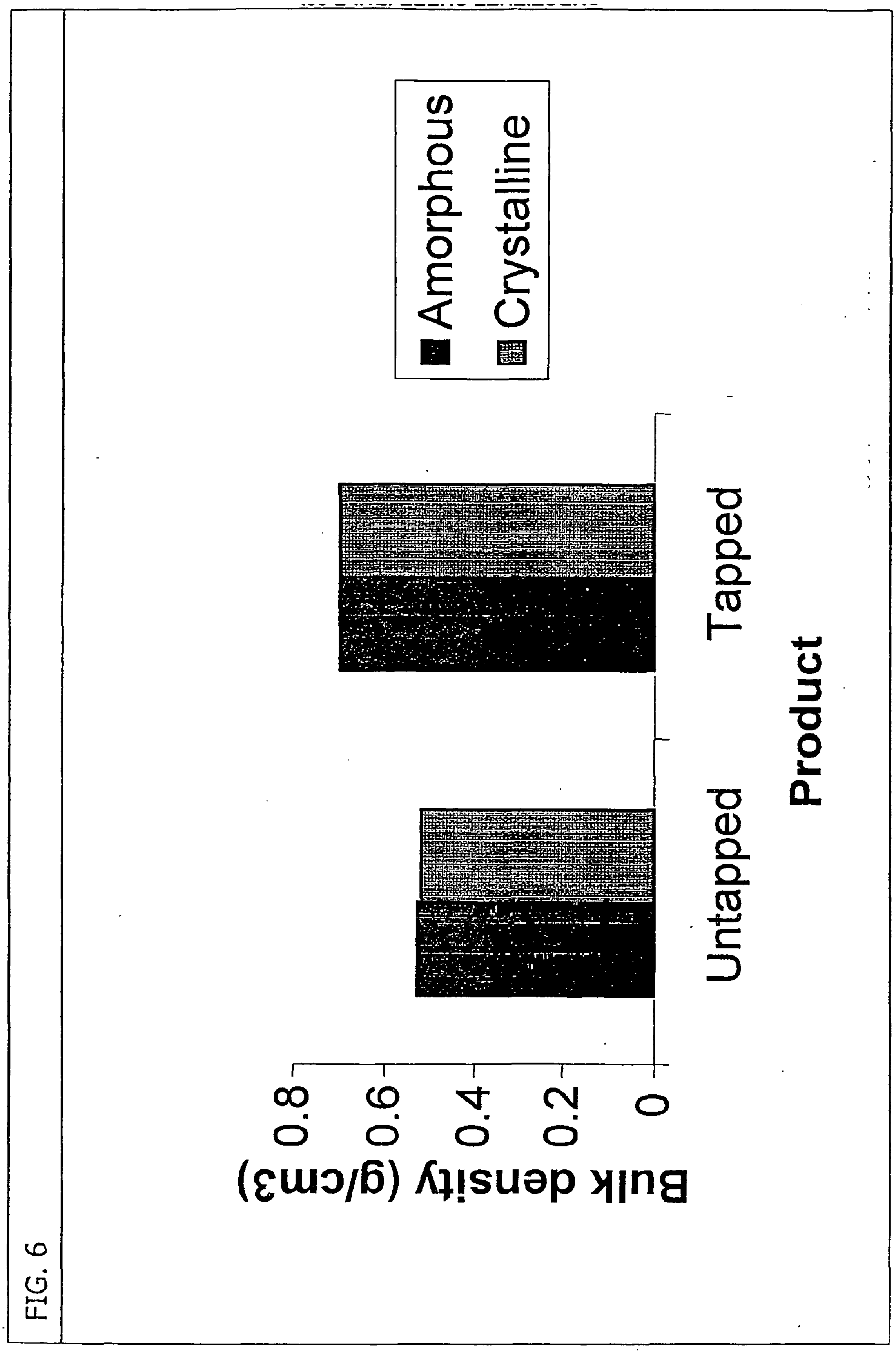

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 63192753 A
- JP 05339238 A
- US 566875 A
- US 09640947 B
- WO 0068229 A
- IN 0000111 W
- US 4399134 A
- US 640947 A

### Non-patent literature cited in the description

- **STEPHEN BYRN.** Pharmaceutical Solids: A strategic Approach to Regulatory Considerations. *Pharma. Res.,* 1995, vol. 12 (7), 945-954